# EUROPEAN PATENT APPLICATION

(11) **EP 2 189 073 A1**
(43) Date of publication of application: **26.05.2010**
(21) Application number: 08831353.1
(22) Date of filing: 17.09.2008
(51) Int. Cl.: A41B 9/12, A41C 1/00

(54) **BELLY BAND**

(30) Priority: 19.09.2007 JP 2007007257 U; 30.10.2007 JP 2007281998
(71) Applicant: Okamoto, Keiji, Hyogo 666-0124 (JP)
(72) Inventor: Okamoto, Keiji, Hyogo 666-0124 (JP)
(74) Representative: Hering, Hartmut
(86) International application number: PCT/JP2008/066730
(87) International publication number: WO 2009/038073

(57) **Abstract**

A belly band that would allow independent stepwise regulation of the degree of tension on each location along the vertical direction of the belly part and would prevent detachment of locking members irrespective of any hard movement during wearing. The belly band (1) comprises a band-shaped cloth (2) having a vertical breadth for covering the belly part and having a length ranging from the perimeter in the waistline direction to twice the same and further not only a male locking part (6) of hook-an-loop fastener attached onto the reverse side of a one-end side of the band-shaped cloth (2) but also, corresponding to the male locking part (6), a female locking part (7) of hook-and-loop fastener attached onto the obverse side of the other-end side of the band-shaped cloth (2) has an attachment width in the waistline direction being larger than that of the male locking part (6) of hook-and-loop fastener. Only the one end of the band-shaped cloth (2) onto which the male locking part (6) of a hook-and-loop fastener is attached is provided with a cutoff area (10) diving the male locking part (6) of hook-and-loop fastener into multiple regions in the vertical breadth direction.

## Description

### Technical field

The present invention relates to an improvement in a belly band which is put around user' s abdomen for use as, for example, a festival costume, an abdomen-waist protector for sporting activities such as golf, and a health-enhancing band for daily use.

### Background Art

A belly band has been used as one of festival costumes ever since before. Such a belly band, which is made for example of a bleached white cotton material having a vertical width of approximately 30 cm and a length of approximately 5 m, is placed around user's abdomen for use. After a cutting work, each end of the bleached material is left as it is and has no locking member or the like. Therefore, for the placement, the bleached material is wrapped around user's abdomen several times with its starting end kept pressed down, and then its terminal end is tucked in the layers of the already-wound material to hold it in place.

However, in the case of holding the material simply by inserting its terminal end between the layers of the material in a wrapped state, due to vigorous physical activities during festivals, the material could sag down easily with the consequence that it needs to be refastened frequently. In this regard various types of belly bands having a locking member at both ends have been proposed to date. For example, one heretofore known belly band employs a hook-and-loop fastener so that it can be fastened in a tension-adjustable manner (refer to Patent documents 1 and 2).
Patent document 1: Japanese Utility Model Registration No. 3065605
Patent document 2: Japanese Unexamined Patent Publication JP-A 8-218204 (1996)

### Disclosure of Invention

### Problems to be solved by the Invention

In the aforementioned construction (Patent document 1), since the locking member of hook-and-loop fastener type is so attached as to extend continuously along the direction of the vertical width of the belly band (vertical direction of abdomen), it follows that a band-retaining tension is regulated uniformly along the vertical direction of abdomen; that is, the belly band cannot be fastened while making stepwise tension regulation at separate locations along the vertical direction of abdomen on an individual basis. Meanwhile, human's abdominal shapes are various and many, for example, there are a drum-like shape, a cylindrical shape, a so-called potbelly, and a conical shape. In the construction employing the lockingmember of hook-and-loop fastener type (Patent document 1), since the dimension of the belly band in its vertical-width direction is approximately 30 cm and thus exceeds a hand-grippable reach (approximately 10 cm), it is impossible to hold the bellyband throughout its overall width at one time. For example, in the case of fastening the belly band with a grip on its midportion in the vertical-width direction, as a part of the hook-and-loop fastener at the midportion is locked, the other part at each of the upper and lower ends of the belly band in the vertical-width direction is also locked dependently without conforming to the shape of user's abdomen. Also in the case of fastening the belly band with a grip on one of its upper and lower ends, the part of the hook-and-loop fastener at the other end as well as the midportion is locked without conforming to the abdominal shape. Even if the user tries to partly release the locking of the hook-and-loop fastener to re-fasten the belly band afterwards, inconveniently, the locking of nearby region is also released unnecessarily. After all, due to the disadvantage that free locking-position adjustment is not possible, the belly band is worn without adjusting to the shape of user's abdomen. For example, the belly band fits snugly over the abdomen at its broadest part but slacks off at its constricted part. In this case, with physical activities during wearing, the belly band could inevitably slip off the abdomen.

By way of contrast, the belly band illustrated in Figs. 1 and 3 of Patent document 2 is so designed that the male locking portion and the female locking portion of hook-and-loop fastener have the same attachment width in the direction of user's waistline. However, this construction is at a disadvantage in that the position of engagement between the male and female locking portions of hook-and-loop fastener cannot be adjustably changed in conformity with user's body shape. That is, if the engagement position is adjustably changed arbitrarily, the area of engagement will be reduced with consequent insufficiency of the force to hold the belly band in a fastened state, which leads to slipping-down of the belly band. In order to avoid such a problem, the belly band needs to be fastened with the area of engagement kept constant. This renders it impossible to adjustably change the engagement position arbitrarily in conformity with user's body shape.

Moreover, in the belly band disclosed in Patent document 2, cutaway portions are formed at both ends of the band-shaped cloth having the male and female locking portions of hook-and-loop fastener, respectively. The cutaway portions at one end and those at the other end are the same in shape and in number. Such a belly band is worn with one end edge of the band-shaped cloth on bottom (skin-side end edge) and the other end edge on top (outer end edge) in an overlapping manner. Therefore, the user has to press the hook-and-loop fastener from above while catching hold of the separate end pieces obtained by creating the cutaway portions. In this case, the hook-and-loop fastener is covered with user' s hand holding the separate end pieces, thus rendering it difficult to establish mutual engagement of hook-and-loop fastener portions at the target end pieces properly. Furthermore, since the separate end pieces are obtained by creating the cutaway portions, some end pieces other than the ones grasped by the hand are folded or dangles automatically, thus hindering the locking action of the hook-and-loop fastener. As another disadvantage, formation of the cutaway portions at both end edges of the band-shaped cloth leads to an undesirable increase in the number of man-hours and to greater cost.

Further, in the belly band disclosed in Patent document 2, it is essential to use, as the band-shaped cloth, a greatly-elasticized knitted material such as knit fabric, particularly a specially-woven knitted material in which knit fibers having high elasticity are arranged in a lattice-like pattern vertically and horizontally of the band-shaped cloth and knit fibers having low elasticity are arranged within the lattice-like pattern. This leads to further cost increase.

The present invention has been devised in view of the aforestated disadvantages associated with the conventional belly bands, and accordingly its object is to provide a belly band that allows stepwise tension regulation at separate locations along a vertical direction of user's abdomen individually and succeeds in preventing accidental detachment of a locking member caused by vigorous physical activities during wearing.

### Means for solving the Problems

In order to accomplish the above object, the present invention provides a belly band composed of: a band-shaped cloth having a vertical width large enough to cover an abdominal region and a length which is greater than the perimeter of the abdominal region in a waistline direction but less than or equal to twice the perimeter; a male locking portion of hook-and-loop fastener attached to a reverse side at one end of the band-shaped cloth; and a female locking portion of hook-and-loop fastener attached, in correspondence with the male locking portion, to an obverse side at the other end of the band-shaped cloth. The female locking portion of hook-and-loop fastener attached to the obverse side at the other end of the band-shaped cloth is larger in attachment width in the waistline direction than the male locking portion of hook-and-loop fastener. Moreover, only at one end of the band-shaped cloth having the male locking portion of hook-and-loop fastener, there are formed cutaway portions to divide the male locking portion of hook-and-loop fastener into a plurality of segments in a vertical-width direction.

In this construction, the male locking portion of hook-and-loop fastener can be adjustably locked in any given position in the waistline direction within the range of attachment width of the female locking portion of hook-and-loop fastener having a larger breadth. At this time, the area of engagement between the male and female locking portions is kept substantially the same regardless of the locking position of the male locking portion, in consequence whereof there results no insufficiency in fastening force for retaining the band-shaped cloth in a wrapped state.

Moreover, the cutaway portions are formed only at one end of the band-shaped cloth that will come on top during wearing (the outer end) ; that is, the other end of the band-shaped cloth that will come on bottom during wearing (the inner end) is devoid of the cutawayportion. Being kept in shape in the vertical-width direction regardless of which location is held by user' s hands, this cutaway-free end will not be folded or dangle, wherefore the separate male locking portion segments of hook-and-loop fastener at the cutaway-bearing end can be bondingly engaged with the female locking portion from above (from outside) without being disturbed by user's hands.

As a matter of course, since the male locking portion of hook-and-loop fastener attached to one end, acting as the outer end, of the band-shaped cloth is formed with the cutaway portions for dividing it into a plurality of segments in the vertical-width direction, it is possible to achieve bonding fastening of the band-shaped cloth in such a manner that the holding position in the vertical-width direction can be adjustably shifted in the waistline direction in conformity with user's body shape. At this time, since the area of engagement between the male and female locking portions of hook-and-loop fastener can be kept constant, it is possible to achieve stepwise tension regulation at separate locations along the vertical direction of the abdominal region individually, as well as to prevent accidental detachment of the locking member caused by vigorous physical activities during wearing.

Moreover, since the cutaway portions are formed only at one end of the band-shaped cloth, it is possible to reduce the number of man-hours and thereby avoid an increase in cost. Another advantage of being able to avoid greater cost is that there is no need to use a specially-woven material for the band-shaped cloth. The cutaway portion may either be formed at one end of the band-shaped cloth in advance or be formed by cutting the band-shaped cloth with scissors afterwards. In the latter case, to allow a purchaser to cut the band-shaped cloth to create the cutaway portions freely after selecting a desired band-shaped cloth fastening position (locking-member locking position) in conformity with his/her body shape, the band-shaped cloth may have formed at its one end a plurality of cutting-aid portions for allowing cutaway formation that are spaced apart along the vertical-width direction.

Moreover, the band-shaped cloth is composed of a front fabric, a lining, and an interlining interposed therebetween stacked on top of one another. In this construction, the interlining imparts tension (rigidity) to the band-shaped cloth, wherefore the band-shaped cloth can be put around user' s abdomen with uniformity in band width in a crinkliness-free state. Further, a protective net having a mail-like structure is detachably inserted between the front fabric and the lining of the band-shaped cloth. In this construction, if there is the possibility of a user getting into danger, the mail-like protective net is interposed between the front fabric and the lining of the band-shaped cloth to protect the user from a stab wound which is made by an edged tool. When unnecessary, the protective net can be removed.

Moreover, in the band-shaped cloth, the front fabric is made of a leather material and the lining is made of a cloth material, and the lining is detachable from the front fabric. In this construction, by virtue of the leather-made front fabric and the cloth-made lining, the belly band succeeds in presenting a fine appearance and in conforming gently to user's skin. Further, being made readily detachable from the front fabric, the lining can be removed freely for cleaning when it soils. Advantageous effect of the Invention

According to the present invention, it is possible to provide a belly band that allows stepwise tension regulation at separate locations along a vertical direction of user' s abdomen individually and succeeds in preventing accidental detachment of a locking member caused by vigorous physical activities during wearing.

### Brief Description of Drawings

[Fig. 1] Figure 1 is a schematic reverse side view of a belly band in a developed state according to the present invention.
[Fig. 2] Figure 2 is a sectional view taken along the line A-A of Fig. 1.
[Fig. 3] Figure 3 is an obverse side view of the belly band shown in Fig. 1.
[Fig. 4] Figure 4 is an enlarged view of a part A shown in Fig. 1.
[Fig. 5] Figure 5 is an enlarged view of a part B shown in Fig. 2.
[Fig. 6] Figure 6 is an enlarged view of a part C shown in Fig. 2.
[Fig. 7] Figure 7 is an enlarged view of a part D shown in Fig. 3.
[Fig. 8] Figure 8 is a schematic reverse side view of the belly band in a developed state according to another embodiment of the present invention.
[Fig. 9] Figure 9 is a schematic reverse side view of the belly band in a developed state according to still another embodiment of the present invention.
[Fig. 10] Figure 10 is a schematic obverse side view of the belly band shown in Fig. 9.
[Fig. 11] Figure 11 is a schematic reverse side view of the main part of yet another embodiment implemented by attaching a covering piece to the end of the belly band of the present invention.
[Fig. 12] Figure 12 is a schematic vertical sectional side view showing the covering piece depicted in Fig. 11 in an unfolded state.
[Fig. 13] Figure 13 is a schematic vertical sectional side view showing the covering piece depicted in Fig. 11 in a state of being folded and tucked in.

### Explanation of reference numerals and symbols

- 1: Belly band
- 2: Band-shaped cloth
- 6: Male locking portion of hook-and-loop fastener
- 7: Female locking portion of hook-and-loop fastener
- 8: Locking member
- 9: Seam in quilted design
- 10: Cutaway portion

### Best Mode for Carrying out the Invention

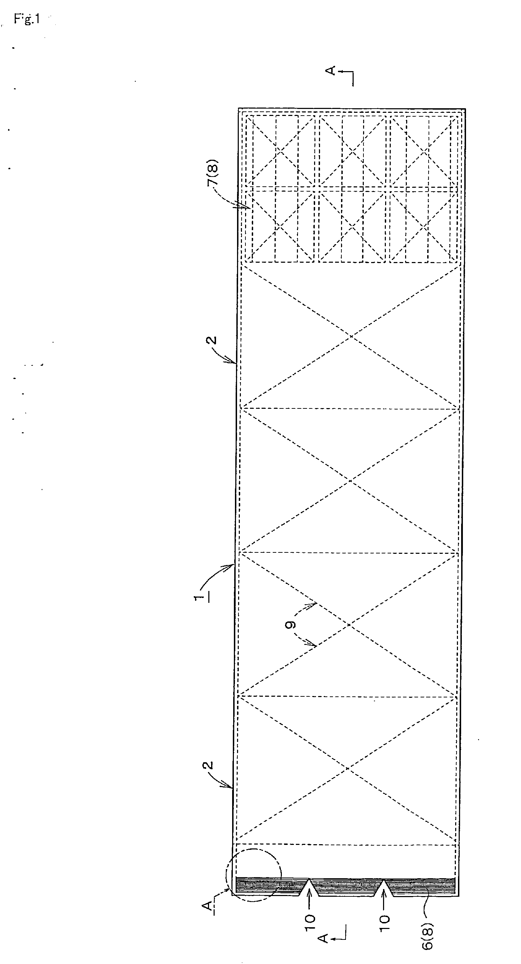

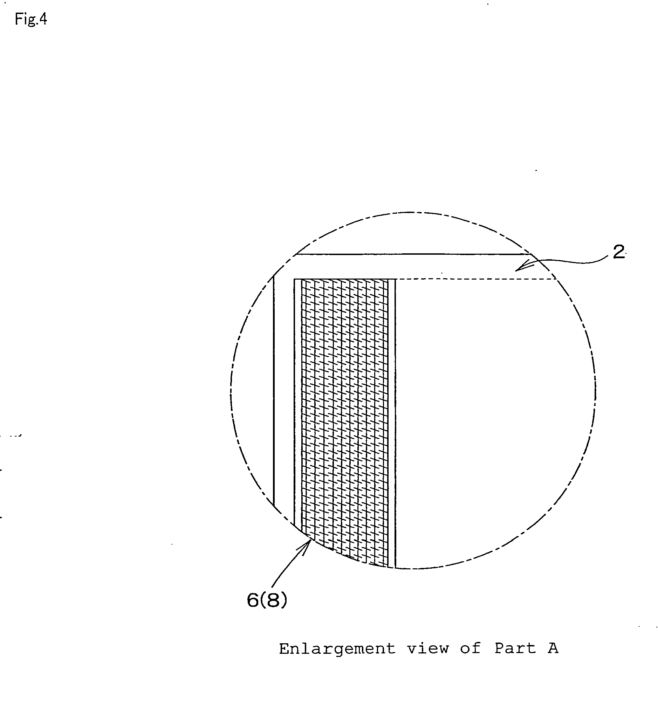

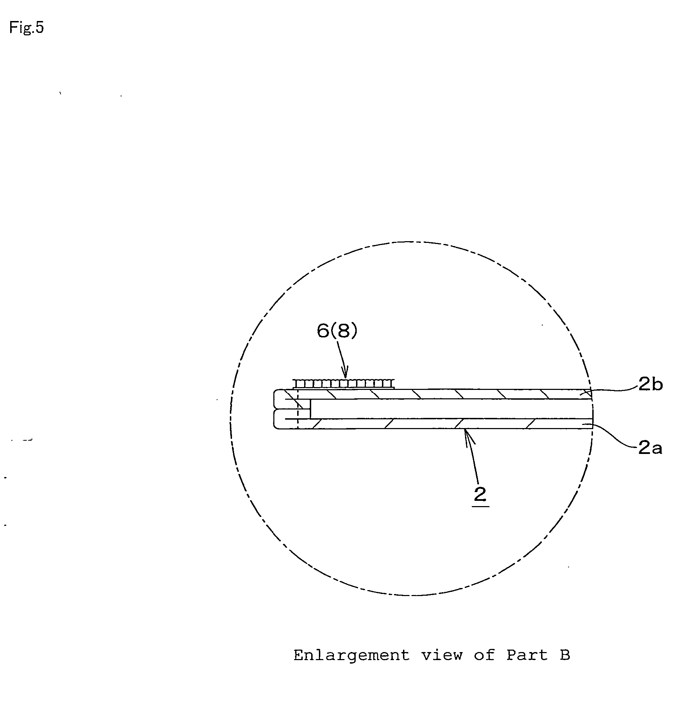

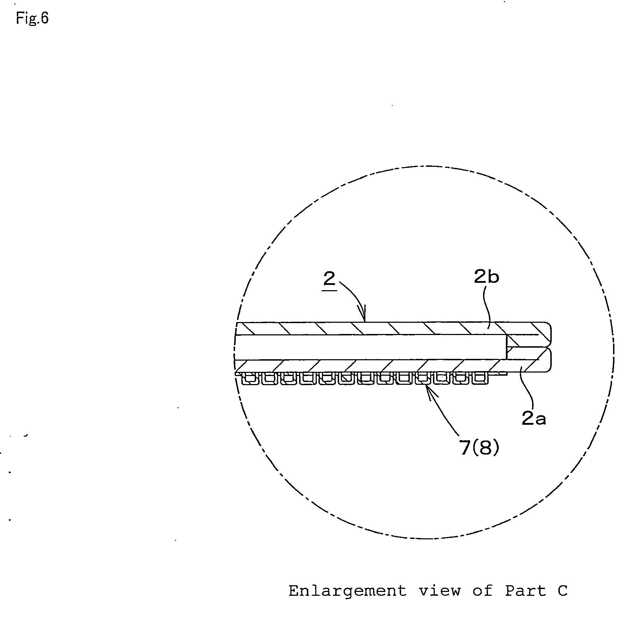

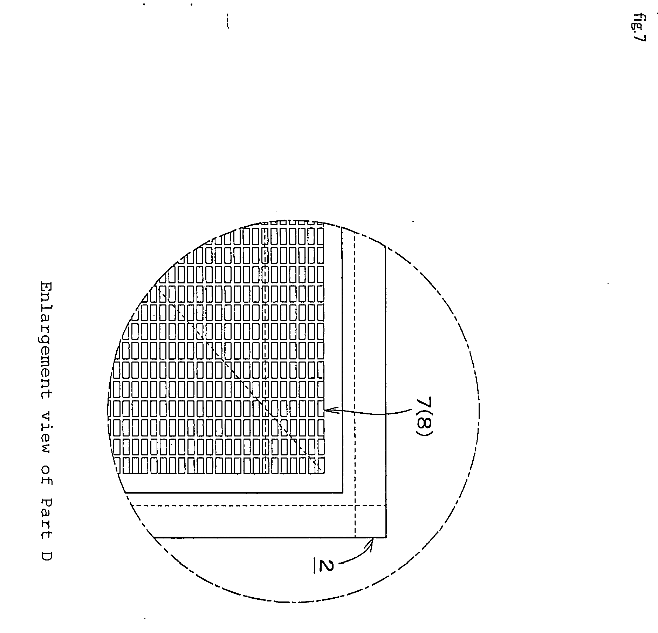

Hereinafter, embodiments of a belly band according to the present invention will be described with reference to the drawings. Figures 1 to 3 are a schematic reverse side view, a A-A sectional view, and an obverse side view, respectively, of a belly band in a developed state of the present invention. Figures 4 to 7 are enlarged views of parts A to D shown in Figs. 1 to 3. As shown in Figs. 1 to 7, a belly band 1 of the present invention is composed of a band-shaped cloth 2 and a locking member 8 constructed of a male locking portion 6 and a female locking portion 7 of hook-and-loop fastener.

The band-shaped cloth 2 has a vertical width large enough to cover an abdominal region and a length which is greater than the perimeter of the abdominal region in a waistline direction but less than or equal to twice the perimeter. For example, the vertical width of the band-shaped cloth 2 is set at approximately 290 mm, and, in order that it may be available in sizes S, M, L, and LL, the length in the waistline direction takes on various values: 63 cm (S), 84 cm (M), 105 cm (L), and 126 cm (LL) (however, the size is not limited thereto and changes may be made arbitrarily). In the hook-and-loop fastener constituting the locking member 8, the male locking portion 6 is attached to a reverse side at one end of the band-shaped cloth 2 by means of sewing or otherwise. Specifically, only in a region toward one end of the band-shaped cloth 2, the male locking portion 6 is disposed so as to extend along substantially the entire length of the band-shaped cloth 2 in its vertical-width direction in the form of a narrow band (for example, ca. 20 mm in width). Note that the vertical length of the male locking portion 6 is set to be somewhat smaller than the vertical width of the band-shaped cloth 2 (for example, the upper, lower extremity of the male locking portion is spaced ca. 10 mm inwardly from the corresponding one of the band-shaped cloth).

Moreover, the male locking portion 6 is spaced by a short distance (for example, ca. 5 mm) away from one end edge of the band-shaped cloth 2 (the left-hand edge as viewed in Fig. 1) toward the other end edge (the right-hand edge as viewed in Fig. 1). In this way, the one end edge of the band-shaped cloth 2 (the left-hand edge as viewed in Fig. 1) protrudes beyond the male locking portion 6 of hook-and-loop fastener. With a grip on this protruding part, engagement and release of the hook-and-loop fastener can be made with ease. In correspondence with the male locking portion 6, the female locking portion 7 of hook-and-loop fastener is attached to an obverse side at the other end of the band-shaped cloth 2 by means of sewing or otherwise.

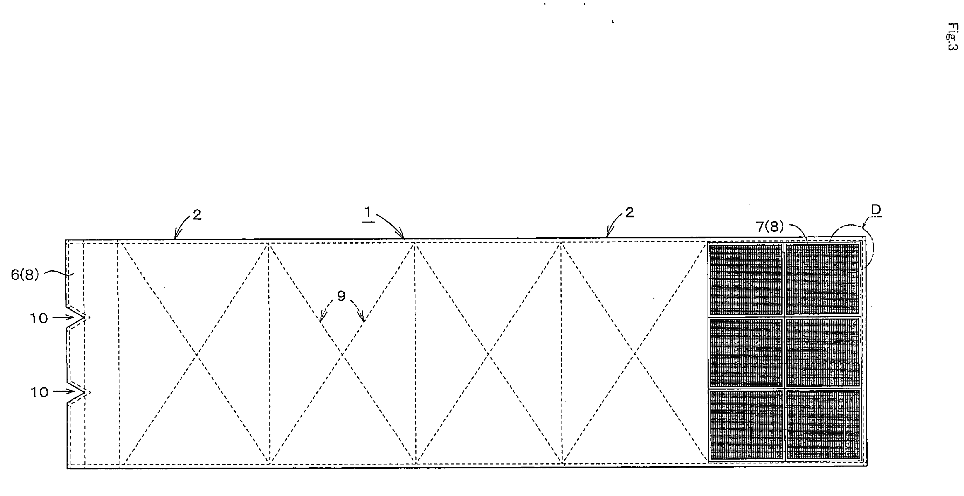

For example, the ca. 200-millimeter-long female locking portion 7 of hook-and-loop fastener is attached so as to extend in the waistline direction. Moreover, the other edge (the right-hand edge as viewed in Fig. 3) of the female locking portion 7 of hook-and-loop fastener is spaced by a short distance (for example, ca. 20 mm) inwardly from the other edge (the right-hand edge as viewed in Fig. 3) of the band-shaped cloth 2. Further, the vertical length of the female locking portion 7 of hook-and-loop fastener is set to be somewhat smaller than the vertical width of the band-shaped cloth 2 (for example, the upper, lower extremity of the female locking portion is spaced ca. 10 mm inwardly from the corresponding one of the band-shaped cloth).

The band-shaped cloth 2 has formed at its one end cutaway portions 10 for dividing the male locking portion 6 of hook-and-loop fastener into a plurality of segments in the vertical-width direction. In this split state, the male locking portion 6 of hook-and-loop fastener can be bondingly engaged with the female locking portion 7 so as to preserve a certain amount of positional freedom in the waistline direction. Accordingly, the band-shaped cloth 2 can be put in a bondingly-fastened state in conformity with user' s body shape.

The cutaway portion 10 may either be formed at one end of the band-shaped cloth 2 in advance or be formed by cutting the band-shaped cloth 2 with scissors afterwards. In the latter case, to allow a purchaser to cut the band-shaped cloth 2 to create the cutaway portions 10 freely after selecting a desired band-shaped cloth fastening position (locking-member locking position) in conformity with his/her body shape, the band-shaped cloth 2 may have formed at its one end a plurality of cutting-aid portions (not represented graphically) for allowing cutaway formation that are spaced apart along the vertical-width direction. The cutting-aid portions may be formed, as cutting-aid lines, on one of or both of the obverse and reverse sides of the band-shaped cloth 2 by means of printing, spraying, textile printing, drawing, or otherwise. Moreover, to prevent appearance of loose threads following the cutting operation, fraying-preventive seams are preferably formed outside the cutting-aid lines so as to be left uncut on the band-shaped cloth 2.

Up to this point the configuration of the belly band 1 according to the embodiment of the present invention has been described, and now its features will be explained. The belly band 1 of the present invention is so designed that the male locking portion 6 of hook-and-loop fastener can be adjustably locked in any given position in the waistline direction within the range of attachment width of the female locking portion 7 of hook-and-loop fastener having a larger breadth. At this time, the area of engagement between the male and female locking portions is kept substantially the same regardless of the locking position of the male locking portion 6, in consequence whereof there results no insufficiency in fastening force for retaining the band-shaped cloth 2 in a wrapped state.

Moreover, the cutaway portions 10 are formed only at one end of the band-shaped cloth 2 that will come on top during wearing (the outer end) ; that is, the other end of the band-shaped cloth 2 that will come on bottom during wearing (the inner end) is devoid of the cutaway portion. Being kept in shape in the vertical-width direction regardless of which location is held by user's hands, this cutaway-free end will not be folded or dangle, wherefore the separate male locking portion segments 6 of hook-and-loop fastener at the cutaway-bearing end can be bondingly engaged with the female locking portion 7 from above (from outside) without being disturbed by user's hands. As a matter of course, since the male locking portion 6 of hook-and-loop fastener attached to one end, acting as the outer end, of the band-shaped cloth 10 is formed with the cutaway portions 10 for dividing it into a plurality of segments in the vertical-width direction, it is possible to achieve bonding fastening of the band-shaped cloth 2 in such a manner that the holding position in the vertical-width direction can be adjustably shifted in the waistline direction in conformity with user's body shape. At this time, since the area of engagement between the male and female locking portions 6 and 7 of hook-and-loop fastener can be kept constant, it is possible to achieve stepwise tension regulation at separate locations along the vertical direction of the abdominal region individually, as well as to prevent accidental detachment of the locking member 8 caused by vigorous physical activities during wearing.

Moreover, since the cutaway portions 10 are formed only at one end of the band-shaped cloth 2, it is possible to reduce the number of man-hours and thereby avoid an increase in cost. Another advantage of being able to avoid greater cost is that there is no need to use a specially-woven material for the band-shaped cloth 2. As described heretofore, according to the present invention, there is provided the belly band 1 that can be fastened with stability while adjusting to the shape of user' s abdomen without causing looseness and slipping-down during wearing.

Moreover, the male locking portion 6 of hook-and-loop fastener constituting the locking member 8 is slightly spaced from one end edge toward the other end edge of the band-shaped cloth 2. Therefore, in effecting bonding engagement and disengagement between the male locking portion 6 of hook-and-loop fastener constituting the locking member 8 and the corresponding female locking portion 7, since the one end edge of the band-shaped cloth 2 protrudes outward beyond the male locking portion 6, with a grip on this protruding part, engagement and release of the hook-and-loop fastener can be readily made without causing any pain in user's fingers.

While the embodiment of the belly band 1 of the present invention has the configuration and features thus far described, the present invention is not limited to the embodiment, and hence some changes and modifications are possible as follows. For example, the back fabric (lining) of the belly band may be made of a material having high moisture absorbency or a breathable material having a silky touch. The belly band may be constructed in sandwich style by inserting a highly hydroscopic material between the lining and the front fabric. The front fabric may be provided with an embroidered design or printed patterns. Although, in the embodiment, the male locking portion 6 and the female locking portion 7 of hook-and-loop fastener constituting the locking member 8 are arranged on the left hand and the right hand, respectively, they may be arranged in the place of each other. Further, the belly band of the present invention may be used as a breast corset for press-holding the breast of a woman wearing a Japanese kimono or taking sporting exercise.

In consideration of women having a large breast size, to prevent their breasts from being subjected to excessive pressure, the band-shaped cloth 2 may be made smaller in vertical width. For example, by setting the vertical width at approximately 220 mm, the upper edge of the band-shaped cloth 2 can be kept clear of the breasts. Moreover, the band-shaped cloth 2 may be formed of any given suitable material. While the band-shaped cloth is, when designed for use as a festival costume for example, generally formed of bleached white cotton cloth, there is no particular limitation and therefore denim (jeans) fabric, woven fabric or knitted fabric of another type, stretchy fabric, and so forth may be used. The band-shaped cloth may also be composed of a front fabric and a lining that differ from each other in cloth composition and color.

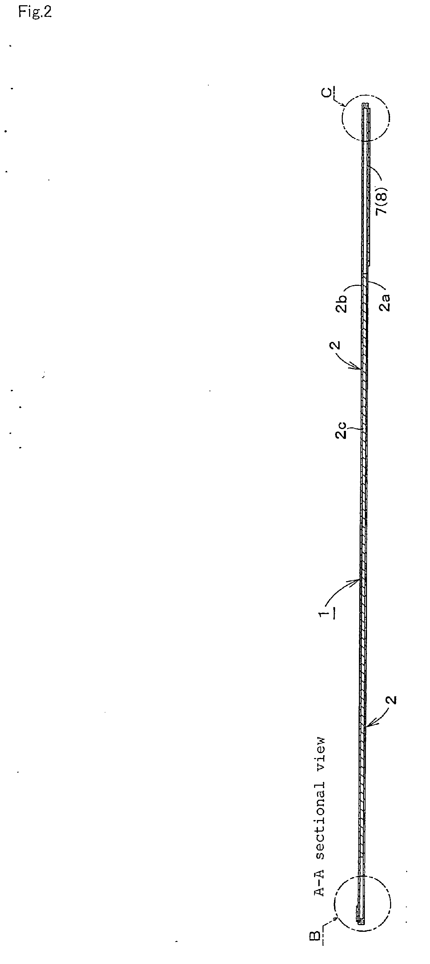

As shown in Fig. 2, in this embodiment, the band-shaped cloth 2 is constructed by inserting an interlining 2c between a front fabric 2a and a back fabric 2b. In this construction, the interlining 2c imparts tension (rigidity) to the band-shaped cloth 2, wherefore the band-shaped cloth 2 can be put around user' s abdomen with uniformity in band width in a crinkliness-free state. Where the range of insertion of the interlining 2c is concerned, the interlining 2c is located away from the lengthwise opposite ends of the band-shaped cloth 2, for example, extends from a point near the attachment position of the male locking portion 6 of hook-and-loop fastener to a point forward of the attachment position of the female locking portion 7 of hook-and-loop fastener. While the belly band 1 of the present invention is illustrated as being formed by sewing the folded-back outer edges of the band-shaped cloth 2 with French seams for prevention of loose threads, it is possible to use instead an edge tape having a U-shaped sectional profile for covering the end edge of the cloth. The front fabric and the back fabric are sewn together, including the range of insertion of the interlining 2c of the band-shaped cloth 2, with seams in quilted design 9.

The band-shaped cloth 2 may be composed of a leather-made front fabric 2a and a back fabric 2b made for example of a sweat-absorbent fabric material such as a cotton cloth. The back fabric 2b may be detachably attached to the leather-made front fabric 2a for cleaning by means of snap-fit buttons, a zipper, or a hook-and-loop fastener. In this construction, by virtue of the leather-made front fabric 2a and the cloth-made back fabric 2b, the belly band 1 succeeds in presenting a fine appearance and in conforming gently to user's skin. Moreover, being made readily detachable from the front fabric 2a, the back fabric 2b can be removed freely for cleaning when it soils. As shown in Figs. 1 and 3, one end of the band-shaped cloth 2 having the male lockingportion 6 of hook-and-loop fastener constituting the locking member 8 is formed with the cutaway portions 10 for dividing the male locking portion 6 of hook-and-loop fastener into a plurality of segments in the vertical-width direction. While the cutaway portions 10 are illustrated as being two in number to divide one end of the band-shaped cloth 2 into three segments in the vertical-width direction, there is no particular limitation. For example, it is possible to provide an additional cutaway portion at the midportion of one end of the band-shaped cloth in the vertical-width direction or to increase the number of the cutaway portions to divide it into four segments. Moreover, while the cutaway portion 10 is illustrated as having the shape of the letter V, it is not limited thereto but may be of another shape.

With the provision of such cutaway portions 10, the separate male locking portion segments 6 of hook-and-loop fastener can be bondingly engaged with the female locking portion 7 so as to preserve a certain amount of positional freedom in the waistline direction. In this case, the cutaway portion 10 is not formed at the other end of the band-shaped cloth 2 that will come on bottom during wearing (the skin-side end) ; that is, the end edge having the female locking portion 7, wherefore this cutaway-free end edge of the band-shaped cloth 2 is kept in shape more securely in the vertical direction and is thus held uprightly simply by pressing part of it by user's one hand. This allows the user to readily bind the band-shaped cloth 2 in conformity with his/her abdominal shape with it held in position by the bonding action of the hook-and-loop fastener. Moreover, the present invention may be implemented as a construction provided with a protective net having a mail-like structure (not represented graphically) that is detachably inserted between the front fabric 2a and the back fabric 2b of the band-shaped cloth 2. In this construction, if there is the possibility of the user getting into danger, the mail-like protective net is interposed between the front fabric 2a and the back fabric 2b of the band-shaped cloth 2 to protect the user from a stab wound which is made by an edged tool. When unnecessary, the protective net can be removed. In this case, it is recommended that U-shaped hooks are disposed at adequate intervals along the lengthwise upper edge on the reverse side of the front fabric 2a. Thereby, the mail-like protective net can be attached to the band-shaped cloth 2 simply by hanging at its upper edge on the hooks.

Moreover, in the present invention, instead of the mail-like protective net, a heat-trapping sheet made of nylon or the like material may be detachably inserted to encourage perspiration during wearing, so that the belly band 1 can be used as a health product for producing weight-loss effect. Also in this case, the heat-trapping sheet is made removable for easy cleaning. Further, the belly band 1 of the present invention may be used as a maternity girdle. In this case, the attachment width of the female locking portion 7 of hook-and-loop fastener is preferably increased so that the perimeter of the belly band in a wrapped state is adjustably increased in the waistline direction in a range, e.g. from 80 to 150 cm with the growth of a baby-to-be.

The belly band 1 of the present invention may be provided with an inside wallet pocket, a cigarette pocket, and an outside small-money pocket. Moreover, the belly band 1 of the present invention may include a pressure point-stimulating member such as magnetic particles or spherical objects for performing finger-pressure treatment on a human body. In this case, the pressure point-stimulating member may be disposed, in the form of buttons or a sheet, in that part of the belly band which abuts against user's back. Further, in the belly band 1 of the present invention, the band-shaped cloth 2 in a developed state may have any given shape, for example, a drum or fan shape, or may be so shaped that its lower edge is curved convexly downwardly.

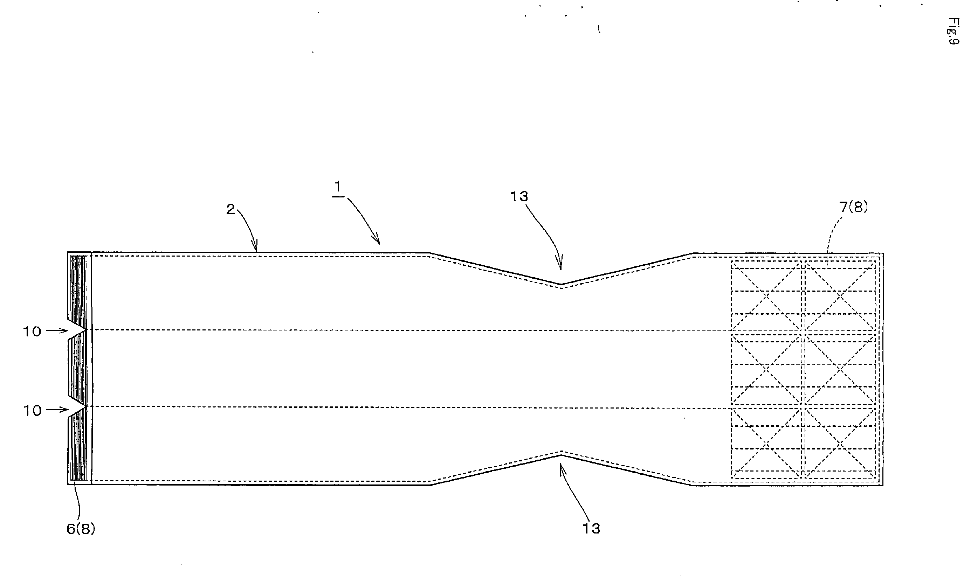

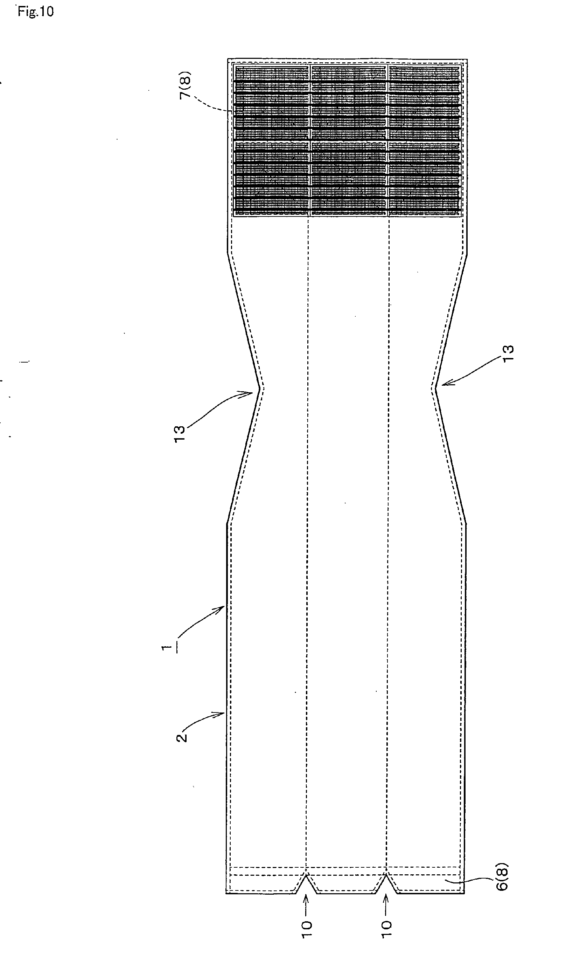

In Figs. 9 and 10, there is shown an example of the band-shaped cloth 2 having V-shaped concavities 13, 13 formed in its upper and lower parts, respectively, so as to lie at a position midway between the opposite ends thereof in the waistline direction. In this case, the V-shaped concavities 13, 13 are preferably formed in that part of the band-shaped cloth which abuts against the central region of user's back during wearing. For example, the length from one end of the band-shaped cloth 2 (the end having the female locking portion 7 of hook-and-loop fastener) to the midpoint of the V-shaped concavity 13 ranges from 320 to 330 mm, and correspondingly the width of the V-shaped concavity 13 in the waistline direction ranges from 340 to 360 mm and the depth of the V-shaped concavity 13 in the vertical-width direction ranges from 35 to 45 mm. In this example, the vertical width of the band-shaped cloth 2 is set at 290 mm, and, in order that it may be available in sizes S, M, L, and LL, the length in the waistline direction takes on various values: 900 mm (S), 1000 mm (M), 1100 mm (L), and 1200 mm (LL). The belly band 1 of the present invention can be used by either right-handed persons or left-handed persons, for example, by being turned upside down.

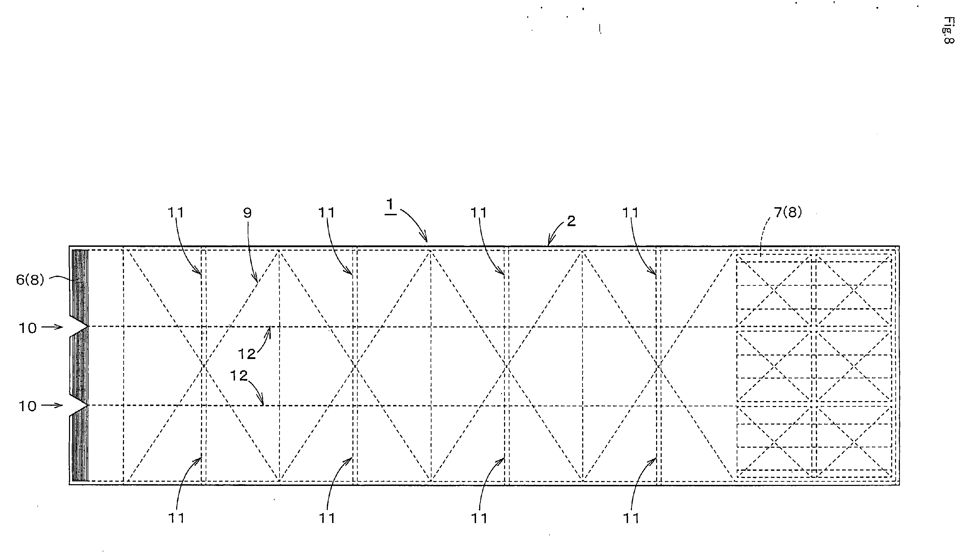

Moreover, as shown in Fig. 8, the belly band 1 of the present invention has, in several positions of the band-shaped cloth 2 in the waistline direction (four positions in the example shown in Fig. 8, but there is no limitation and the number of positions may be either increased or decreased), slit-forming seams 11 formed so as to extend along the entire length of the band-shaped cloth 2 in the vertical-width direction. The slit-forming seams 11 located at different positions are each made in the form of two parallel lines. A slit is formed by cutting the region between the two lines of the seam 11 with scissors or the like, wherefore appearance of loose threads around the slit can be prevented after the cutting operation. With such a slit, it is possible to let out the hem side of the band-shaped cloth 2 and thereby allow the belly band to fit snugly to the body of a user whose figure is such that the abdomen or waist is relatively small in size but the perimeter in the waistline direction increases gradually with increasing proximity to the hips (for example, women). The purchaser is able to make a slit at any given position in conformity with his/her body shape. The slit is formed so as to extend from the lower side (hem side) of the band-shaped cloth 2 to a point spaced therefrom by a distance of about one-third the vertical width toward the upper side- not to extend along the entire length of the band-shaped cloth 2 in the vertical-width direction. As a guide for making the slit, seams 12, 12 perpendicular to the seams 11 are formed in a location spaced a distance of about one-third the vertical width toward the lower side from the upper side and a location spaced a distance of about one-third the vertical width toward the upper side from the lower side, respectively, so as to extend in the waistline direction. In order that the belly band may be used by either right-handed persons or left-handed persons by being turned upside down, the seam 11 extends along the entire length of the band-shaped cloth in the vertical-width direction, and the seams 12, 12 are arranged vertically in a distance of 1/3 the vertical width one from another. The information about the slit-cutting operation is borne, along with illustrations, in an explanatory leaflet placed in a package of the belly band 1 of the present invention, so that the purchaser is able to use the article in the best condition.

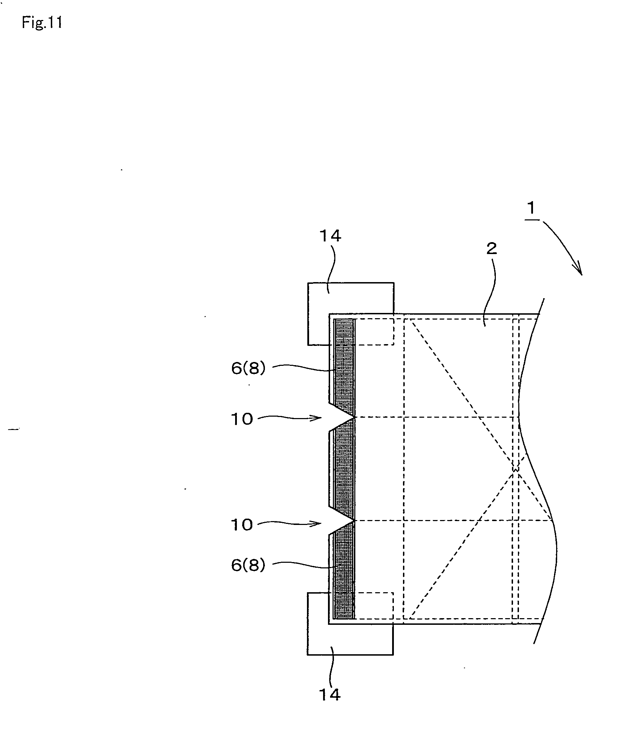

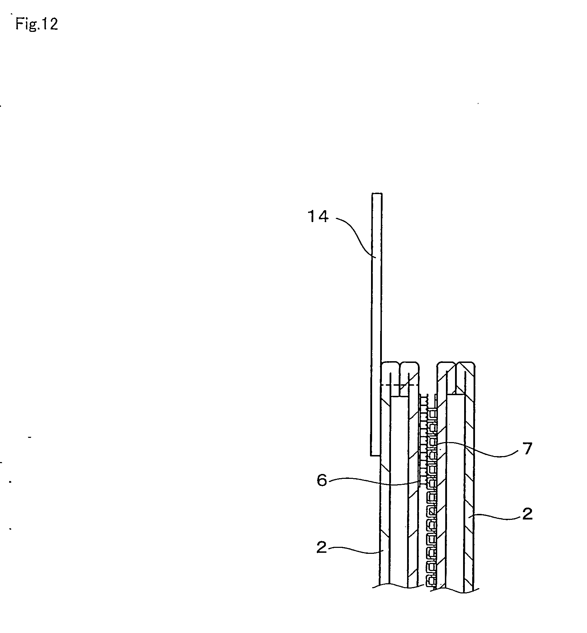

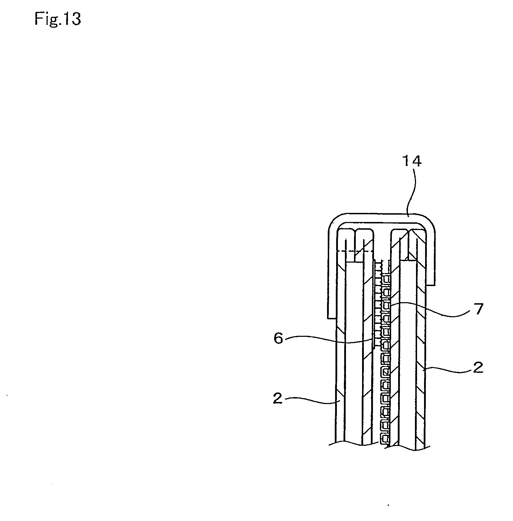

Moreover, as shown in Fig. 11, cloth-made covering pieces 14, 14 may be sewn on the top and the bottom, respectively, of one end of the band-shaped cloth 2 having the male locking portion 6 of hook-and-loop fastener so as to protrude beyond the top and the bottom, respectively. During placement of the band-shaped cloth 2 around user's abdomen, as shown in Fig. 12, the covering pieces 14, 14 are kept in a vertically-upright state. Following the completion of binding, as shown in Fig. 13, the covering pieces 14, 14 are tucked in the inner (skin-side) part of the band-shaped cloth. Thereby, even if the male locking portion 6 of hook-and-loop fastener, which will come on top when the band-shaped cloth 2 is put around user's abdomen (the outer part), protrudes upward or downward beyond the female locking portion 7, since the covering pieces 14, 14 help prevent direct contact between the protruding part and user's skin, it is possible to provide cloth softness and thereby protect the user from contact pain during wearing. The attachment width of the covering piece 14 is equal to or slightly larger than the attachment width of the male locking portion 6 of hook-and-loop fastener in the waistline direction. For example, the width of the covering piece 14 in the waistline direction is set at approximately 60 mm, and the length of its vertical protrusion is set at approximately 30 mm. Industrial Applicability

The belly band of the present invention is used as a festival costume, an abdomen-waist protector for sporting activities such as golf, a health-enhancing band for daily use, a weight-loss band, a body-shape control band, a maternity girdle for abdomen protection, and so forth.

## Claims

1. A belly band comprising:
a band-shaped cloth (2) having a vertical width large enough to cover an abdominal region and a length which is greater than the perimeter of the abdominal region in a waistline direction but less than or equal to twice the perimeter;
a male locking portion (6) of hook-and-loop fastener attached to a reverse side at one end of the band-shaped cloth (2); and
a female locking portion (7) of hook-and-loop fastener attached, in correspondence with the male locking portion, to an obverse side at the other end of the band-shaped cloth (2),
wherein the female locking portion (7) of hook-and-loop fastener attached to the obverse side at the other end of the band-shaped cloth (2) is larger in attachment width in the waistline direction than the male locking portion (6) of hook-and-loop fastener,
and wherein, only at one end of the band-shaped cloth (2) having the male locking portion (6) of hook-and-loop fastener, there are formed cutaway portions to (10) divide the male locking portion (6) of hook-and-loop fastener into a plurality of segments in a vertical-width direction.

2. A belly band comprising:
a band-shaped cloth (2) having a vertical width large enough to cover an abdominal region and a length which is greater than the perimeter of the abdominal region in a waistline direction but less than or equal to twice the perimeter;
a male locking portion (6) of hook-and-loop fastener attached to a reverse side at one end of the band-shaped cloth; (2) and
a female locking portion (7) of hook-and-loop fastener attached, in correspondence with the male locking portion, to an obverse side at the other end of the band-shaped cloth (2)
wherein the female locking portion (7) of hook-and-loop fastener attached to the obverse side at the other end of the band-shaped cloth (2) is larger in attachment width in the waistline direction than the male locking portion (6) of hook-and-loop fastener,
and wherein, only at one end of the band-shaped cloth (2) having the male locking portion (6) of hook-and-loop fastener, there are formed cutting-aid portions (10) for allowing formation of cutaway portions to divide the male locking portion (6) of hook-and-loop fastener into a plurality of segments in a vertical-width direction.

3. The belly band as set forth in claim 1 or 2,
wherein the band-shaped cloth (2) is composed of a front fabric, a lining, and an interlining interposed therebetween stacked on top of one another.

4. The belly band as set forth in claim 3,
wherein a protective net having a mail-like structure is detachably inserted between the front fabric and the lining of the band-shaped cloth (2).

5. The belly band as set forth in claim 3,
wherein, in the band-shaped cloth (2), the front fabric is made of a leather material and the lining is made of a cloth material, and the lining is detachable from the front fabric.

6. The belly band as set forth in claim 4,
wherein, in the band-shaped cloth (2), the front fabric is made of a leather material and the lining is made of a cloth material, and the lining is detachable from the front fabric.
